Europäisches Patentamt

European Patent Office

Office européen des brevets

(11) Publication number: **0 415 470 B1**

# EUROPEAN PATENT SPECIFICATION

(45) Date of publication of patent specification: **04.01.95**

(51) Int. Cl.⁶: **C12N 9/52**, C12N 1/21, C12P 21/08, C12N 15/57, C12Q 1/37, A23L 1/03, C12P 21/00, A23C 19/032, A23L 1/314, //(C12N9/52, C12R1:225)

(21) Application number: **90202098.1**

(22) Date of filing: **01.08.90**

(54) **Aminopeptidase.**

(30) Priority: **02.08.89 NL 8901994**

(43) Date of publication of application:
**06.03.91 Bulletin 91/10**

(45) Publication of the grant of the patent:
**04.01.95 Bulletin 95/01**

(84) Designated Contracting States:
**AT BE CH DE DK ES FR GB GR IT LI LU NL SE**

(56) References cited:
**EP-A- 0 286 171**

**APPLIED MICROBIOLOGY AND BIOTECH-NOLOGY, vol. 31, no. 1, July 1989, pages 75-78,Springer-Verlag; B. KIEFER-PARTSCH et al.: "Purification of an X-prolyl-dipeptidyl aminopeptidase from the cell wall proteolytic system of Lactococcus lactic subsp. cremoris"**

(73) Proprietor: **DMV CAMPINA B.V.**
**NCB-laan 80**
**NL-5462 GE Veghel (NL)**

Proprietor: **Rijksuniversiteit te Groningen**
**Broerstraat 5**
**NL-9712 CP Groningen (NL)**

(72) Inventor: **Tan, Paris Som Tjwan**
**Vinkenstraat 122a**
**NI-9713 TM Groningen (NL)**
Inventor: **Konings, Wilhelmus Nicolaas**
**Rijksstraatweg 236**
**NI-9752 CJ Haren (NL)**
Inventor: **Levering-Clement, Wendy**
**Plecht 13**
**NL-5345 BT Oss (NL)**
Inventor: **Zuurendonk, Peter Frederik**
**Notelaar 8**
**NL-5467 BB Veghel (NL)**

FEMS MICROBIOLOGY REVIEWS, vol. 46, 1987, pp. 245-268, Elsevier, Amsterdam, NL; T.D. THOMAS et al.: "Proteolytic enzymes of dairy starter cultures"

APPLIED AND ENVIRONMENTAL MICROBI-OLOGY, vol. 56, no. 2, February 1990, pages 526-532, American Society for Microbiology; P.S.T. TAN et al.: "Purification and character-ization of an aminopeptidase from Lactococ-cus lactis subsp.cremoris Wg2"

(74) Representative: **Smulders, Theodorus A.H.J., Ir. et al**
**Vereenigde Octrooibureaux**
**Nieuwe Parklaan 97**
**NL-2587 BN 's-Gravenhage (NL)**

**Description**

This invention relates to an aminopeptidase of lactic acid bacteria in isolated form, i.e. in a substantially pure state in relation to the state in which it is present in lactic acid bateria in vivo.

The invention further relates to transformed organisms, especially microorganisms (bacteria, but also fungi and yeasts) capable of expression of the relative aminopeptidase (i.e. capable of forming the aminopeptidase); to a recombinant polynucleotide, more in particular recombinant DNA, which contains the genetic information coding for the aminopeptidase, and to the use of such a recombinant polynucleotide as a transformation marker; to a process for preparing the aminopeptidase through isolation from a crude cell-free extract of an organism in which the aminopeptidase is expressed; to the use of the aminopeptidase for preparing polyclonal and monoclonal antibodies against the aminopeptidase (i.e with affinity and/or specificity for the aminopeptidase), as well as to such antibodies as such and to their use for detecting, removing and/or isolating the aminopeptidase; to a process for preparing food products, such as cheese and other dairy products, meat products and protein hydrolysates, in which the aminopeptidase is used, and to the thus obtained food products.

Lactic acid bacteria, especially lactic acid lactococci such as Lactococcus lactis subsp. cremoris, lactis and diacetylactis, and other lactic acid bacteria such as Leuconostoc species are widely used in the food industry, especially in the dairy industry for preparing fermented milk products such as cheese. The production of lactic acid from lactose results in acidification of the food product so as to prevent the food product from spoilage. Moreover, the bacteria are involved in the development of taste and smell of the food product. Here an important process is the degradation of proteins.

For growth and for lactic acid production the bacteria need a nutrient medium providing essential growth factors such as vitamins and amino acids. Milk is a suitable nutrient medium and enables growth up to high cell densities. To this end, however, the content of free amino acids is insufficient in milk so that as far as their need for amino acids is concerned the bacteria depend on their proteolytic system for the degradation of milk proteins, especially casein. The proteolytic system of lactic acid bacteria remains active also after termination of the growth, e.g., in the cheese ripening phase in which the degradation of casein to peptides and then amino acids contributes to the aroma and taste development of the cheese.

The proteolytic system of lactic acid lactococci (also referred to as lactic acid streptococci; the species Lactococcus lactis subsp. cremoris is identical with Streptococcus lactis subsp. cremoris or Streptococcus cremoris) comprises different proteases and different peptidases. The proteases are localised in the cell wall and within the cell of the bacteria, while the peptidases can be present in the medium, in the cell wall and within the cell.

Up to now only a few peptidases of lactococci have been isolated and characterised. Hwang et al, Agric. Biol. Chem. 45, 1981, 159-165, and 46, 1982, 3049-3053, have isolated a dipeptidase with a broad substrate specificity from Lactococcus lactis subspecies cremoris H61. Geiss et al, Appl. Microbiol. Biotechnol. 23, 1985, 79-84, have extracted and purified a cell wall bound aminopeptidase of S. cremoris AC$_1$. Desmazeaud and Zevaco, Milchwissenschaft 34, 1979, 606-610, have isolated two intracellular, not yet characterised aminopeptidases of Lactococcus lactis subspecies diacetylactis.

Exterkate et al, Appl. Environ. Microbiol. 15, 1987, 577-583, have isolated an extracellular aminopeptidase from Lactococcus lactis subsp. cremoris. Kaminogawa et al, Agric. Biol. Chem. 46, 1984, 3035-3040, have isolated a prolidase from Lactococcus lactis subsp. cremoris H61. Yan et al, Eur. J. Biochem. 163, 1987, 259-265 and Appl. Environ. Microbiol. 53, 1987, 2296-2302, have isolated two new endopeptidases from Lactococcus lactis subsp. cremoris H61. Van Boven et al, Appl. Environ. Microbiol. 54, 1988, 43-49, have isolated a dipeptidase from Lactococcus lactis subsp. cremoris Wg2.

In the Dutch cheese industry Lactococcus lactis subspecies cremoris is the most important lactic acid bacterial species. A good insight into the proteolytic system of lactic acid lactococci such as Lactococcus lactis subspecies cremoris can contribute to an improvement of the starter cultures and to a better regulation of the ripening process, e.g., resulting in an acceleration of cheese ripening or prevention of a bitter taste (formation of bitter peptides). Much basic information is given in the dissertation by R. Otto entitled: "An ecophysiological study of starter streptococci", State University of Groningen (1981) and in the dissertation by J. Hugenholtz: "Population dynamics of mixed starter cultures", State University of Groningen (1986). The latter dissertation and the dissertation by A. van Boven entitled: "The uptake and hydrolysis of peptides by S. cremoris", describe a further investigation into the proteolytic system of Lactococcus lactis subspecies cremoris Wg2.

The present invention is based on a further investigation into the peptidases of Lactococcus lactis subspecies cremoris Wg2, in which a specific aminopeptidase that had not been described before was isolated and characterised. The aminopeptidase was purified from a crude cell-free extract of Lactococcus lactis subsp. cremoris Wg2 by DEAE-Sephacel column chromatography, followed by phenyl-Sepharose chromatography, gel filtration and anion exchange chromatography (HPLC). The aminopeptidase exhibits hydrolysis activity with respect to several peptides and is, in view of its sensitivity to the chelating compounds EDTA and 1,10-phenanthroline, a metallo-enzyme exhibiting a pH optimum at about 7 and a temperature optimum at about 40°C. By SDS-PAA gel electrophoresis of the purified enzyme a single protein band is found having a molecular weight of about 95 kD. The enzyme experiences strong inhibition of compounds such as p-chloromercuribenzoate (pCMB), p-chloromercuribenzenesulphonate (pCMBS), O-(3-hydroxymercuri-2-methoxypropyl) carbamyl phenoxyacetate (mersalyl), N-ethylmaleimide (NEM) and iodoacetamide capable of blocking sulphydryl groups and is insensitive to disulphide bonds-reducing agents such as $\beta$-mercaptoethanol and dithiotreitol and to phenylarsine oxide and PMSF. A kinetic investigation into the lysine-p-nitroanilide (lys-pNA) hydrolysis shows that the enzyme possesses a relatively low affinity for this substrate (Km 0.55 mM), but a very high hydrolysis velocity maximum ($V_{max}$ 1000 $\mu$mol/min. mg protein). This value corresponds to a turnover number of about 1580 $sec^{-1}$. The pI (isoelectric point) of the enzyme is about 6.2.

The novel aminopeptidase according to the invention differs from the aminopeptidase isolated by Geiss et al from Lactococcus lactis subsp.cremoris $AC_1$ in molecular weight and in reactivity with metal ions. It differs in substrate specificity from the dipeptidases earlier described by Hwang et al and Van Boven et al and from the aminopeptidase isolated by Exterkate et al from Lactococcus lactis subsp. cremoris HP in molecular weight, substrate specificity, reactivity with divalent metal ions and activity in the presence of DTT and $\beta$-mercaptoethanol.

The novel aminopeptidase hydrolyses several peptides, including leu-leu, leu-gly, glu-ala, gly-lys, ile-ala, leu-gly-gly, $leu_3$, ala-val-leu, ala-pro-ala, ala-pro-phe, ala-his-ala, ala-pro-gly, gly-pro-ala, $ala_3$, $ala_4$, $ala_5$, lys-pNA, leu-pNA, met-enkephalin and bradykinin. On the other hand, the novel aminopeptidase does not prove capable of significant hydrolysis of dipeptides such as ala-glu, ala-gly, ala-ala, phe-leu and phe-val, containing ala and phe as N-terminal amino acid, and tripeptides containing N-terminal gly-gly.

The novel aminopeptidase according to the invention has in common with the earlier described known peptidases of lactic acid lactococci that the chelating o-phenanthroline gives a strong inhibition of the hydrolysis activity, which activity can then be restored by adding specific divalent cations such as $Zn^{++}$ and $Co^{++}$. Surprisingly, however, higher concentrations of $Zn^{++}$ are found to give an inhibitory effect.

The novel aminopeptidase could be used to avoid development of an undesirable bitter taste during cheese ripening. The fact is that after degradation of casein some Lactococcus lactis subsp. cremoris strains are found to produce different degrees of bitterness, apparently as a result of accumulation of peptides having a high content of hydrophobic amino acids which are broken down only slowly by the peptidases of the bacteria contained in the starter. The use of the present aminopeptidase could mitigate this problem.

The aminopeptidase could also be used for other purposes such as the clarification of beer and soft drinks, and it could of course also be used in DNA technology in the broadest sense.

The invention first resides in the aminopeptidase itself, i.e. an aminopeptidase of lactic acid bacteria in isolated form, characterised by a molecular weight of 95 kD ± 5 kD; an isoelectric point of 6.2 ± 0.4; a substrate range comprising various dipeptides, tripeptides and higher, including leu-leu, leu-gly, glu-ala, gly-lys, leu-gly-gly, leu-leu-leu, ala-val-leu, gly-leu-tyr, ala-ala-ala, ala-ala-ala-ala, tyr-gly-gly-phe-met, ala-ala-ala-ala, and ala-ala-ala-ala-ala-ala, but not ala-ala, ala-glu, and ala-gly; a hydrolysis activity with respect to lysyl-p-nitroanilide with a temperature optimum at 40°C ± 5°C and a pH optimum at 7 ± 0.5; a sensitivity to both chelating agents EDTA and 1,10-phenanthroline and to the sulphydryl blockers p-chloromercuribenzoate, p-chloromercuribenzenesulphonate, and O-(3-hydroxymercuri-2-methoxypropyl)-carbamylphenoxyacetate.

The aminopeptidase according to the invention can further be characterised by the following amino acid sequence at the N-terminal end: ala-val-lys-arg-leu-ile-glu-thr-phe-val-pro-glu-asn-tyr, more in particular by the following amino acid sequence at the N-terminal end: ala-val-lys-arg-leu-ile-glu-thr-phe-val-pro-glu-asn-tyr-lys-ile-phe-leu-asp-ile-asp-arg-lys, and even more in particular by the following amino acid sequence at the N-terminal end: ala-val-lys-arg-leu-ile-glu-thr-phe-val-pro-glu-asn-tyr-lys-ile-phe-leu-asp-ile-asp-arg-lys-X-thr-lys-ile-lys-gly-gln-val-ala-ile, in which X is a not yet determined amino acid.

Although the aminopeptidase which according to the experimental part is virtually isolated and characterised originates from one specific organism, namely a proteinase negative variant of Lactococcus lactis subsp. cremoris Wg2, the invention also comprises the corresponding aminopeptidases of other lactic acid bacteria since these possess corresponding properties and can be isolated from the bacteria and purified in a corresponding manner. By "corresponding aminopeptidases" is not meant that the peptidases are exactly the same, i.e. have the same amino acid sequence, but is rather meant that they satisfy the above-mentioned characterisation of the aminopeptidase.

A more specific embodiment of the invention is concerned with the aminopeptidase originating from lactic acid lactococci, in particular Lactococcus lactis subsp. cremoris, most preferably Lactococcus lactis subsp. cremoris Wg2.

The words "in isolated form" and "originating from" do not mean to limit the invention to aminopeptidase isolated from its natural environment. In fact, the invention creates the possibility of tracing the aminopeptidase gene and then realising production of the aminopeptidase in transformed cells or organisms by means of recombinant DNA technology. For this purpose, e.g., the amino acid sequence of the aminopeptidase could first be determined, or part thereof (as has been done in the present case). On the basis thereof, corresponding DNA sequences could be derived by means of the genetic code, after which suitable DNA probes can be synthesised, with which aminopeptidase messenger RNA (mRNA) of the cells can be detected and isolated. This mRNA can be used according to known per se techniques for preparing copy DNA (cDNA) using enzymes such as reverse transcriptase and DNA polymerase. This cDNA can then be cloned by means of suitable vectors (mostly plasmids) and expressed in suitable host cells, from which the aminopeptidase produced can be easily recovered.

The aminopeptidase according to the invention can therefore be obtained by its isolation from a cell mass, a culture medium or a separation product of lactic acid bacteria, in which the aminopeptidase is naturally expressed, or from a cell mass, a culture medium or a separation product of an organism which, by means of genetic engineering, has acquired the power to express the aminopeptidase.

The invention further comprises a process for preparing an aminopeptidase according to the invention, which process is characterised in that a crude cell-free extract of an organism, in particular a microorganism, in which the aminopeptidase is expressed, is subjected to one or more ion exchange column chromatography and gel filtration steps, in which of each eluate collected in fractions one or more of the fractions having hydrolysis activity with respect to lysyl-, leucyl- or methionyl-p-nitroanilide are selected.

The invention also creates the possibility of producing antibodies against the aminopeptidase and then using them to remove or isolate the aminopeptidase from its natural environment or from the above transformed cells or organisms.

Of course, the invention further resides in a process for preparing food products such as cheese and other dairy products, meat products and protein hydrolysates, using an aminopeptidase and/or an organism according to the invention. Specific hydrolysis properties of the aminopeptidase may be utilised here, e.g., to inhibit development of a bitter aroma or to realise accelerated cheese ripening. The process may also have the advantage of lower production cost, e.g., because less starter is required.

The invention will hereinafter be further illustrated with reference to the experimental part.

## Materials and methods

### Organism and preparation of a cell-free extract

In the experiments a proteinase-negative variant of Lactococcus lactis subsp. cremoris Wg2 was used. The organism was stored by routine in 10% (wt./vol.) sterile reconstituted skim milk containing 0.1% (wt./vol) tryptone at a temperature of -20°C. For culturing Lactococcus lactis subsp. cremoris Wg2, MRS medium (De Man et al, J. Appl. Bacteriol. 23, 1960, 130-135) was used with the growth occurring at a controlled pH of 6.3 in a 5 l fermenter. Cells were harvested at an $A_{660nm}$ of 0.8, washed in 200 ml 0.05 M potassium phosphate (pH 7) and suspended again in 50 ml of this buffer. Cells were broken by sonication (soniprep 150; MSE Scientific Instruments, Crawley, England) for 480 sec. (8x 15 sec. sonication and 45 sec. rest) at 10 micrometer at 4°C under a constant nitrogen stream. By centrifugation of the broken cells for 10 min. at 20,000 g and 4°C, cell extract was obtained.

### Enzyme determinations

Hydrolysis of lysyl-p-nitroanilide was determined by the method of Exterkate, Neth. Milk Diary J. 29, 1975, 303-318. A 2 mM solution of lysyl-p-nitroanilide was incubated for 15 minutes with a suitable amount of enzyme. The reaction was stopped by adding acetic acid up to a final concentration of 10%, and the amount of p-nitroanilide was measured at 410 nm (Hitachi U-1100 spectrophotometer).

All measurements of enzyme activities were carried out at 30°C in 20 mM HEPES pH 7, unless specified.

Hydrolysis of several peptides was detected by thin-layer chromatography (TLC). Peptidase activity was determined as follows: the reaction mixture containing 2 mM substrate in 20 mM 4-(2-hydroxyethyl)-1-piperazine-ethanesulphonic acid (HEPES) pH 7 and a suitable amount of enzyme was incubated for 60 min. at 30°C. The reaction mixture (10 microliter) was then placed on a precoated silica gel 60 plate having a layer thickness of 0.25 cm (Merck, Darmstadt, West-Germany). A 75% (wt./vol.) phenol/water mixture and in some cases a 4/1/1 (vol./vol./vol.) mixture of n-butanol, acetic acid and water was used as mobile phase. As a control, 2 mM of each standard peptide was also placed on the plate. The silica gels were coloured by spraying them with 0.1% (wt./vol.) ninhydrin in 99% ethanol. Peptides and amino acids became visible after incubation of the silica gel in an oven for 5 min. at 80°C.

DEAE-Sephacel column chromatography

A DEAE-Sephacel column (1.6 x 20 cm) was equilibrated with 10 mM $K_2HPO_4/KH_2PO_4$ pH 7.0 with 0.12 M NaCl. The cell extract obtained after sonication and centrifugation of L. lactis subsp. cremoris Wg2 was diluted with distilled water to the same ion strength as the buffer used for equilibrating the DEAE-Sephacel column and was then placed on the column. After washing the column with 2 volume amounts of equilibration buffer, the enzyme was eluted (40 ml/hour) with a linear gradient of 0.12 M to 0.4 M NaCl in the same buffer. Fractions of 3 ml were collected and tested for lysyl-p-nitroanilide hydrolysis activity. The fractions having the highest enzyme activity were combined.

Phenyl Sepharose chromatopgraphy

A phenyl-Sepharose (CL-4B) column (1.8 x 8.5 cm) was equilibrated with 10 mM $K_2HPO_4-KH_2PO_4$ pH 7 containing 4 M NaCl. NaCl was added to the combined enzyme fractions to the same molarity as the equilibration buffer, and the enyme solution was placed on the column. After washing the column with 2 volume amounts of equilibration buffer, the enzyme was eluted (35 ml/hour) with a linear gradient of 4 M NaCl to 0 M NaCl in 10 mM $K_2HPO_4-KH_2PO_4$ pH 7. Fractions of 2.5 ml were collected and tested for lysyl-p-nitroanilide hydrolysis activity. Fractions having the highest enzyme activity were combined.

Gel chromatography on Sephadex G-100 Super Fine

The enzyme fractions obtained from the phenyl-Sepharose CL-4B column were concentrated in an Amicon filtration unit with PM-10 and YM-10 membranes (with a limit at a molecular weight of 10,000; Amicon Corp., Lexington, Mass.) to a final volume of about 2 ml and then placed on a Sephadex G-100 SF column equilibrated with 10 mM $K_2HPO_4-KH_2PO_4$ (pH 7).containing 0.1 M NaCl. Elution was effected with the same buffer at a flow rate of 4 ml/hour, and portions of 2 ml were collected. Fractions having the highest peptidase activities were combined.

High-performance liquid chromatography: anion exchange chromatography

The combined fractions obtained from the gel filtration were concentrated again to a volume amount of 2.5 ml. At each step 400 microliter enzyme was placed on an HRLC MA7P anion exchange column (50x7.8 mm; Bio-Rad Lab. Richmond, USA) by injecting 4x 100 microliter at intervals of 2 minutes in a constant stream of equilibration buffer of 20 mM Tris/HCl (pH 7.5) containing 125 mM NaCl. The column was washed with 2 ml of the same buffer, and the enzyme activity was eluted with a linear gradient of 0.125 M to 0.4 M NaCl in 20 mM Tris/HCl pH 7.5. The flow rate was 1 ml/min., and peak fractions were collected and tested for peptidase activity. HPLC was carried out with a Waters 600 Multisolvent Delivery System with a U6K universal liquid chromatography injector, and $A_{280nm}$ was measured with a lambda Max model 481 LC spectrophotometer (Millipore, Waters Associates, Milford, Mass., USA).

SDS-PAGE

Sodium dodecyl sulphate (SDS) polyacrylamide gel electrophoresis (PAGE) was carried out as described by Laemmli and Faure, J. Mol. Biol. 80, 1973, 575-599. The protein samples were mixed 4 : 1 with sample buffer (0.45 M Tris-HCl pH 6.8, 0.75% SDS, 22% glycerol, 2.5% $\beta$-mercaptoethanol, and 0.18% bromophenol blue) and placed on the gels.

The molecular weight of the enzymes was estimated by means of the following reference proteins: myosin (200 kD), beta-galactosidase (116.2 kD), phosphorylase b (97.4 kD), bovine serum albumin (66.2 kD), ovalbumin (42.7 kD), bovine carbonic anhydrase (31 kD), soya bean trypsin inhibitor (21.5 kD), and lysozyme (14.4 kD).

Silver colouring of the gels was carried out according to Wray et al, Anal. Biochem. 118, 1981, 197-203.

Isoelectric focusing

For isoelectric focusing (IEF) on slab gels a Pharmacia Phast System with a ready-made 5% PAA gel containing pharmalyte 3-10 (Phastgel; Pharmacia) was used. The isoelectric point of the enzyme was determined using the following references: lentil lectin basic (pI 8.65), lentil lectin medium (pI 8 45), lentil lectin acid (pI 8.15), myoglobin basic (pI 7.35), human carbonic anhydrase B (pI 6.55), bovine carbonic anhydrase (pI 5.85), beta-lactoglobulin A (pI 5.2). The gels were automatically coloured according to the Phast System with Coomassie brilliant blue.

Effect of divalent cations and chemical substances on the enzyme activity

Purified enzyme (15 $\mu$g/ml) was incubated for 10 min. at 20°C with 200 $\mu$M 1,10-phenanthroline in 20 mM N-2-hydroxyethyl-piperazine-N'-2-morpholinoethanesulphonic acid (HEPES) pH 7. After equilibration in a cuvette for 5 min. at 30°C, 1 mM lysyl-p-nitroanilide was added. Several concentrations of divalent cations were added, and the enzyme activity was measured at the release of p-nitroanilide, which was spectrophotometrically monitored at 410 nm in a Hitachi 1100 spectrophotometer.

The effects of chemical substances were determined by incubating 300 $\mu$l of a suitable enzyme solution (15 $\mu$g/ml) for 10 min. at 20°C with different concentrations of different chemical substances.

The enzyme activity was determined by the above method.

Reactivation of the enzyme activity by reducing agents

Purified enzyme (15 $\mu$g/ml) was preincubated with 5 $\mu$M pCMB or 5$\mu$M mersalyl for 10 min. at 20°C. A part of the treated enzyme was incubated again for 10 min. at 20°C with 100 $\mu$M dithiotreitol (DTT) or 100 $\mu$M $\beta$-mercaptoethanol, before lysyl-p-nitroanilide was added. The enzyme activity was determined (15 min. 30°C) at the release of p-nitroanilide and was spectrophotometrically measured at 410 nm.

Determination of the amino acid sequence at the N-terminus of the protein.

The protein sequence was determined by means of the Edman degradation method using an Applied Biosystems model 477A pulse liquid sequencer. Samples were prepared by electroblotting on polyvinylidene difluoride (PVDF) membranes according to Matsudaira, J. Biol. Chem. 262, 1987, 10035-10038; and after colouring with Coomassie blue the amino acid sequence was immediately determined.

Protein determination

Protein concentrations were determined by the method of Lowry et al., J. Biol. Chem. 193, 1951, 265-275, by means of bovine serum albumin as a standard.

Chemicals

All chemicals used were commercially available reagent grade substances.

RESULTS

By different methods an aminopeptidase of Lactococcus lactis subsp. cremoris Wg2 could be purified to homogeneity. A rapid and easy procedure consisted of the following steps.

Enzyme purification

Step a.

The crude cell-free extract was placed on a DEAE-Sephacel column. After elution with an NaCl gradient lysyl-p-nitroanilide hydrolysis activities were obtained in different fractions at 0.18-0.2 M NaCl.

Step b.

The active fractions were combined, 4 M NaCl was added, after which the enzyme solution was placed on a phenyl-Sepharose column. After elution with a reverse NaCl gradient (4 M-0 M) the activity with respect to lysyl-p-nitroanilide was found in a peak eluted at 50 mM NaCl.

Step c.

The aminopeptidase-containing fractions were combined and concentrated in a filtration unit with a PM-10 and YM-10 membrane (with a limit at a molecular weight of 10,000; Amicon Corporation, Lexington, Mass.) to a volume of 2.1 ml. Subsequently, 0.5 ml glycerol was added, and the sample was further purified over a G-100 SF gel filtration column.

Step d.

For the last step the combined aminopeptidase fractions were concentrated to a volume of 2.6 ml and placed on an HPLC MA7P anion exchange column (Bio Rad). The pure enzyme was eluted at 0.35 M NaCl in Tris/HCl (pH 7.5) at a retention time of 13-15 min. The results of the enzyme purification are summarised in Table A. By means of this procedure the enzyme could be purified about 32-fold in 12% yield from the crude extract.

Molecular weight and isoelectric point

On SDS-PAGE the purified enzyme gave only one band, even in the presence of $\beta$-mercaptoethanol. The molecular weight was estimated at 95,000. The same result was obtained under denaturing and native conditions, which indicates that the enzyme is likely to be a monomer consisting of one subunit.

The pI of the enzyme was estimated at 6.2 by isoelectric focusing on a Pharmacia Phast System 5% PAA gel containing pharmalyte 3-10.

Temperature dependence and effect of the pH on the enzyme activity

The effect of the temperature on the aminopeptidase activity was measured in the range of from 5 to 80°C. The enzyme mixture was equilibrated for 5 min. at the test temperatures before lysyl-p-nitroanilide was added. The optimum temperature for the lysyl-p NA-hydrolysis activity was found to be 40°C. At 55°C the activity measured was only 10% of the optimum activity.

The effect of the pH was determined in the pH range of from 4 to 10 using a buffer consisting of 20 mM of each of the substances malic acid, 2-(N-morpholino)ethanesulphonic acid (MES), HEPES and boric acid and was adjusted at the suitable pH value with potassium hydroxide. The optimum pH for hydrolysis of lysyl-PNA proved to be a pH of 7. At values below pH 5 and above pH 9 no hydrolysis activity of the enzyme could be detected.

Substrate specificity

The specificity of the aminopeptidase relative to several peptides is summarized in Table B. The hydrolysis products were analysed by thin-layer chromatography (TLC). The enzyme was active with respect to several peptides, but not with respect to dipeptides containing proline, analine or phenylaniline at the N-terminus. A hydrolysis activity of several peptides could also be observed, but not with tripeptides containing proline or glycine-glycine at the N-terminus.

In contrast to the hydrolysis activity with respect to dipeptides with N-terminal analine, the aminopeptidase was found to be capable of hydrolysis of tripeptides with N-terminal aniline, which indicates that the enzyme has more affinity for oligopeptides than for dipeptides. This assumption is supported by the observation that the enzyme is active with respect to different polyanilines, but not with respect to alanyl-alanine.

The enzyme can also act on different large biologically active peptides such as met-enkaphalin and bradykinin, but does not seem capable of hydrolysis of specific carboxypeptidase substrates such as benzoyl-glycyl-lysine, carboxybenzoxy-phenylalanyl-alanine, and carbobenzoxy-prolylalanine. Table C shows the relative hydrolysis velocities through the enzyme of different substrates with C-terminal p-nitroanilide. The highest activity was found for lysyl-p-nitroanilide and to a lesser extent for leucyl-p-nitroanilide. This observation indicates that this enzyme is an aminopeptidase.

The affinity of the enzyme for lysyl-p-nitroanilide was determined. The Lineweaver-Burk plot indicates a Km for lysyl-pNA of 0.55 mM with a maximum peptide hydrolysis velocity of 1 millimol/min./mg.

## Effect of metal ions on the enzyme activity

Treatment of the enzyme with the chelating agents EDTA and 1,10-phenanthroline led to a complete inhibition of the enzyme activity. The activity of the enzyme treated with 1,10-phenanthroline could be restored to 88% and 80% by addition of 50 $\mu$M $ZnCl_2$ and 50 $\mu$M $CoCl_2$, respectively. Zinc ions proved to be the most effective stimulator, but at concentrations of more than 100 $\mu$M they led to inhibition. Other metal ions such as $Cu^{++}$, $Ca^{++}$, $Mg^{++}$, $Mn^{++}$, and $Fe^{++}$ could not restore the enzyme activity. On the other hand, the activity could not be restored with any of the divalent cations when the enzyme was treated with EDTA.

The inhibitory effect of different divalent cations on the enzyme activity was also examined. Additions of 25 $\mu$M $CuCl_2$ and 25 $\mu$M $Cd(NO_3)_2$ gave an inhibition of the activity of 8% and 35%, respectively. $ZnCl_2$ and $FeCl_2$ (100 $\mu$M) also gave inhibition of the enzyme activity, but to a lesser extent of 32% and 60%, respectively. Divalent calcium, magnesium, and manganese ions had no inhibitory effect at all. All metal ions except $Cd^{++}$ were added in the form of chlorides to prevent any effect of the anions. The results indicate that the aminopeptidase activity depends on the divalent cations $Zn^{++}$ and $Co^{++}$.

## Effects of several chemical reagents

The effects on the aminopeptidase activity of different reagents to sulphydryl groups and thiol groups were examined. The aminopeptidase activity was completely inhibited by the sulphydryl groups-blocking reagents p-chloromercuribenzoate (pCMB), p-chloromercuribenzenesulphonate (p-CMBS) and O-(3-hydroxymercuri-2-methoxypropyl)carbamylphenoxyacetate (mersalyl). Sulphydryl group reagents such as iodoacetamide and N-ethylmaleimide (NEM) also had an inhibitory effect on the enzyme activity, but only at higher temperatures.

Phenylarsine oxide, a dithiol reagent, had no effect on the hydrolysis activity of the enzyme at 2.5 mM. Disulphide-reducing agents such as dithiotreitol (DTT) and $\beta$-mercaptoethanol also had no inhibitory effect on the enzyme activity. These results suggest that the hydrolysis activity depends on one or more reactive SH groups in the protein. This was confirmed by experiments in which the inactivating effects of pCMB and mersalyl were completely undone by DTT or $\beta$-mercaptoethanol. The results are summarised in Table D.

## Determination of the amino acid sequence at the N-terminus of the peptidase

Table E shows the sequence of the N-terminal part of the purified enzyme. Sequence determination was carried out with a pulse liquid ("gaseous phase") sequencer. Up to now 33 amino acid residues could be analysed, the underlined amino acids of which are not yet quite certain.

## Test of debittering activity

To test the capacity of the enzyme to reduce the bitterness in protein hydrolysates, the following experiment was done.

A hydrolysate of caseinate was prepared by treating a sodium caseinate (pH 6.8) with a pancreatic enzyme preparation (Corolase PP, Röhm) until a degree of hydrolysis of 10% was reached. After inactivation of the enzyme by heating, the solution was freeze-dried.

1 gram of the freeze-dried powder was suspended in 10 ml of water. This solution was perceived as intensely bitter. An amount of the aminopeptidase, which is capable of splitting 10-40 $\mu$moles/min of lysyl-p-nitroanilide, was added to the hydrolysate, and after an incubation time of 5-10 hours at 30°C, the bitterness of the solution was no longer evident, at least it was significantly reduced to give a relatively neutral tasting solution.

Table A

| Purification of a lysyl-p-nitroanilide aminopeptidase of L. lactis subsp. cremoris Wg2 | | | | | |
|---|---|---|---|---|---|
| Purification step | Total protein (mg) | Total* activity $10^3$ | Yield (%) | Specific activity ** | Purification (single) |
| Cell extract | 904 | 108 | 100 | 120 | 1 |
| DEAE-Sephacel | 81.8 | 65.6 | 61 | 800 | 6.6 |
| Phenyl-Seph. | 27.4 | 35.7 | 33 | 1300 | 10.8 |
| G-100 SF. | 15.4 | 21.5 | 20 | 1400 | 11.6 |
| MA7P (HPLC) | 3.3 | 12.7 | 12 | 3900 | 31.6 |

* ) Total activity is expressed as $\mu$mol p-nitroanilide released per minute

** ) Specific activity is expressed as $\mu$mol p-nitroanilide released per mg protein per minute

Table B: Substrate specificity of an aminopeptidase of
L.lactis subsp. cremoris Wg2. Hydrolysis of peptides
was analysed by thin-layer chromatography (TLC)

| Substrate | Activity | Substrate | Activity |
|---|---|---|---|
| Leu-Leu | + | Leu-Gly-Gly | + |
| Leu-Gly | + | Leu-Leu-Leu | + |
| Ala-Glu | − | Ala-Val-Leu | + |
| Ala-Gly | − | Ala-Pro-Ala | + |
| Ala-Ala | − | Ala-Pro-Phe | + |
| Glu-Val | − | Ala-His-Ala | + |
| Glu-Ala | + | Ala-Pro-Gly | + |
| Gly-Lys | + | Glu-Val-Phe | − |
| Ile-Ala | + | Gly-Pro-Ala | + |
| Phe-Leu | − | Gly-Leu-Tyr | + |
| Phe-Val | − | Gly-Phe-Leu | + |
| Pro-Met | − | Gly-Ser-Ala | − |
| | | Gly-Gly-Leu | − |
| $Ala_2$ | − | Gly-Gly-Phe | − |
| $Ala_3$ | + | Gly-Gly-Gly | − |
| $Ala_4$ | + | Pro-Gly-Gly | − |
| $Ala_5$ | + | Phe-Gly-Gly | − |
| $Ala_6$ | + | Gly-Pro-Gly-Gly | − |
| Met-enkaphalin | + | Neurotensine | + |
| Bradykinin | + | Glucagon | + |
| Substance P | + | | |

+ : hydrolysis

− : no hydrolysis to be observed

Table C

| Relative activity of the peptidase with respect to several X-p-nitroanilide peptides | | | |
|---|---|---|---|
| Substrate | Relative acitivity (%) | Substrate | Relative activity (%) |
| Lys-pNA | 100 | Phe-pNA | 4 |
| Leu-pNA | 24 | Gly-pNA | < 1 |
| Met-pNA | 8 | | |
| Glu-pNA | < 1 | Gly-Arg-pNA | < 1 |
| Ala-pNA | 4 | Ala-Pro-pNA | 4 |
| Pro-pNA | < 1 | Ala-Ala-Ala-pNA | < 1 |

Table D: Effect of pCMB/DTT and mersalyl/ß-mercaptoethanol on the peptidase activity.
Purified enzyme (15 µg/ml) was preincubated with 5 µM pCMB or 5 µM mersalyl for 10 min. at 20°C.
A part of the treated enzyme was incubated again for 10 min. at 20°C with 100 µM dithiotreitol (DTT) or 100 µM ß-mercaptoethanol before adding lysyl-p-nitroanilide.
Release of p-nitroanilide was spectrophotometrically monitored at 410 nm.

| Additions | % activity |
|---|---|
| no additions | 100 |
| 5 µM pCMB | 29 |
| 5 µM pCMB + 100 µM DTT | 105 |
| 5 µM mersalyl | 34 |
| 5 µM mersalyl + 100 µM ß-merc.eth. | 110 |

Table E: The N-terminal amino acid sequence of the purified
aminopeptidase of L.lactis subsp. cremoris Wg2

```
 1    2    3    4    5    6    7    8    9   10   11   12   13   14   15
Ala-Val-Lys-Arg-Leu-Ile-Glu-Thr-Phe-Val-Pro-Glu-Asn-Tyr-Lys
16   17   18   19   20   21   22   23   24   25   26   27   28   29   30
Ile-Phe-Leu-Asp-Ile-Asp-Arg-Lys- ? -Thr-Lys-Ile-Lys-Gly-Gln-
31   32   33
Val-Ala-Ile......
```

**Claims**

1. An aminopeptidase of lactic acid bacteria in isolated form, characterised by a molecular weight of 95 kD ± 5 kD; an isoelectric point of 6.2 ± 0.4; a substrate range comprising various dipeptides, tripeptides and higher, including leu-leu, leu-gly, glu-ala, gly-lys, leu-gly-gly, leu-leu-leu, ala-val-leu, gly-leu-tyr, ala-ala-ala, ala-ala-ala-ala (Seq. Id. No. 1), tyr-gly-gly-phe-met (Seq. Id. No. 2), ala-ala-ala-ala-ala (Seq. Id. No. 3), and ala-ala-ala-ala-ala-ala (Seq. Id. No. 4), but not ala-ala, ala-glu, and ala-gly; a hydrolysis activity with respect to lysyl-p-nitroanilide with a temperature optimum at $40\,^\circ C \pm 5\,^\circ C$ and a pH optimum at 7 ± 0.5; sensitivity both to chelating agents EDTA and 1,10-phenanthroline and to the sulphydryl blockers p-chloromercuribenzoate, p-chloromercuribenzenesulphonate, and O-(3-hydroxymercuri-2-methoxypropyl)carbamylphenoxyacetate, and the following amino acid sequence at the N-terminal end: ala-val-lys-arg-leu-ile-glu-thr-phe-val-pro-glu-asn-tyr (Seq. Id. No. 5).

2. An aminopeptidase according to claim 1, characterised by the following amino acid sequence at the N-terminal end: ala-val-lys-arg-leu-ile-glu-thr-phe-val-pro-glu-asn-tyr-lys-ile-phe-leu-asp-ile-asp-arg-lys (Seq. Id. No. 6).

3. An aminopeptidase according to claim 1, characterised by the following amino acid sequence at the N-terminal end: ala-val-lys-arg-leu-ile-glu-thr-phe-val-pro-glu-asn-tyr-lys-ile-phe-leu-asp-ile-asp-arg-lys-Xaa-thr-lys-ile-lys-gly-gln-val-ala-ile (Seq. Id. No. 7), in which Xaa is a not yet determined amino acid.

4. An aminopeptidase according to any of claims 1-3, originating from lactic acid lactococci.

5. An aminopeptidase according to claim 4, originating from the lactic acid lactococcus Lactococcus lactis.

6. An aminopeptidase according to claim 4, originating from the lactic acid lactococcus Lactococcus lactis subsp. cremoris.

7. An aminopeptidase according to claim 4, originating from the lactic acid lactococcus Lactococcus lactis subsp. cremoris Wg2.

8. An aminopeptidase according to any of claims 1-7, obtained by its isolation from a cell mass, a culture medium or a separation product of lactic acid bacteria in which the aminopeptidase is naturally expressed, or from a cell mass, a culture medium or a separation product of an organism which, by means of genetic engineering, has acquired the power to express the aminopeptidase.

9. A process for preparing an aminopeptidase according to any of claims 1-8, characterised in that a crude cell-free extract of an organism, in particular a microorganism, in which the aminopeptidase is expressed, is subjected to one or more ion exchange column chromatography and gel filtration steps, in which of each eluate collected in fractions one or more of the fractions having hydrolysis activity with respect to lysyl-p-nitroanilide are selected.

10. The use of an aminopeptidase according to any of claims 1-8 for preparing polyclonal or monoclonal antibodies having affinity and/or specificity for the aminopeptidase.

11. A process for preparing food products such as cheese and other dairy products, meat products and protein hydrolysates, characterised by using an aminopeptidase according to any of claims 1-8.

**Patentansprüche**

1. Aminopeptidase von Milchsäurebakterien in isolierter Form, gekennzeichnet durch ein Molekulargewicht von 95 kD ± 5 kD; einen isoelektrischen Punkt von 6,2 ± 0,4; einen Substratbereich, der verschiedene Dipeptide, Tripeptide und höhere einschließlich leu-leu, leu-gly, glu-ala, gly-lys, leu-gly-gly, leu-leu-leu, ala-val-leu, gly-leu-tyr, ala-ala-ala, ala-ala-ala-ala (Seq. Id. Nr. 1), tyr-gly-gly-phe-met (Seq. Id, Nr. 2), ala-ala-ala-ala-ala (Seq. Id. Nr. 3), und ala-ala-ala-ala-ala-ala (Seq. Id, Nr. 4), jedoch nicht ala-ala, ala-glu, und ala-gly umfaßt; eine Hydrolyse-Aktivität bezüglich Lsyl-p-nitroanilid mit einem Temperaturoptimum bei 40°C ± 5°C und einem pH-Optimum bei 7 ± 0,5; eine Empfindlichkeit gegenüber Chelatbildern EDTA und 1,10 -Phenanthrolin und gegenüber dem Sulphydryl Blockern p-Chloromercuribenzoat, p-Chloromercuribenzensulphonat, und 0-(3-Hydroxymercuri-2-Methoxypropyl)carbamylphenoxyacetat, und der folgenden Aminosäuresequenz am N-Ende:
ala-val-lys-arg-leu-ile-glu-thr-phe-val-pro-glu-asn-tyr (Seq. Id, Nr. 6).

2. Aminopeptidase nach Anspruch 1, gekennzeichnet durch die folgende Aminosäuresequenz am N-Ende:
ala-val-lys-arg-leu-ile-glu-thr-phe-val-pro-glu-asn-tyr-lys-ile-phe-leu-asp-ile-asp-arg-lys (Seq. Id, Nr. 6).

3. Aminopeptidase nach Anspruch 1, gekennzeichnet durch die folgende Aminosäuresequenz am N-Ende:
ala-val-lys-arg-leu-ile-glu-thr-phe-val-pro-glu-asn-tyr-lys-ile-phe-leu-asp-ile-asp-arg-lys-Xaa-thr-lys-ile-lys-gly-gln-val-ala-ile (Seq. Id, Nr. 7), wobei Xaa eine noch nicht bestimmte Aminosäure ist.

4. Aminopeptidase nach einem der Ansprüche 1 bis 3, die aus Milchsäurelactococci stammt.

5. Aminopeptidase nach Anspruch 4, die aus Milchsäure-Lactococcus <u>Lactococcus</u> <u>lactis</u> stammt.

6. Aminopeptidase nach Anspruch 4, die aus Milchsäure-Lactococcus <u>Lactococcus</u> <u>lactis</u> subsp. cremoris stammt.

7. Aminopeptidase nach Anspruch 4, die aus Milchsäure-Lactococcus <u>Lactococcus</u> <u>lactis</u> subsp. <u>cremoris</u> Wg2 stammt.

8. Aminopeptidase nach einem der Ansprüche 1 bis 7, die durch ihre Isolation aus einer Zellmasse, einem Kulturmedium oder einem Trennprodukt aus Milchsäurebakterien erhalten wird, worin die Aminopeptidase natürlich ausgepreßt wird, oder von einer Zellmasse, einem Kulturmedium oder einem Trennprodukt eines Organismus erhalten wird, der durch Gentechnik die Kraft zum Auspressen der Aminopeptidase gewonnen hat.

9. Verfahren zur Herstellung einer Aminopeptidase nach einem der Ansprüche 1 bis 8, dadurch gekennzeichnet, daß eines rohes zellfreies Extrakt aus einem Organismus, insbesondere einem Mikroorganismus, in welchem die Aminopeptidase ausgepreßt ist, einer oder mehreren Ionenaustauschersäulen-Chromatographie und Gel-filtrationsstufen unterworfen wird, wobei in jedem in Fraktionen gesammelten Eluat eine oder mehrere der Fraktionen, die Hydrolyseaktivität bezüglich Lysyl-p-Nitroanilid haben, ausgewählt werden.

10. Verwendung einer Aminopeptidase nach einem der Ansprüche 1 bis 8 zur Herstellung von polyclonalen oder monoclonalen Antikörpern mit einer Affinität und/oder spezifischen Eigenschaft für die Aminopeptidase.

**11.** Verfahren zur Herstellung von Nahrungsmitteln wie etwa Käse und anderen Milchprodukten, Fleischprodukten und Proteinhydrolysaten, gekennzeichnet durch die Verwendung einer Aminopeptidase nach einem der Ansprüche 1 bis 8.

**Revendications**

**1.** Aminopeptidase de bactéries lactiques sous forme isolée, caractérisée par un poids moléculaire de 95 kD ± 5 kD ; un point isoélectrique de 6,2 ± 0,4 ; une gamme de substrats comprenant divers dipeptides, tripeptides et des peptides supérieurs, comprenant leu-leu, leu-gly, glu-ala, gly-lys, leu-gly-gly, leu-leu-leu, ala-val-leu, gly-leu-tyr, ala-ala-ala, ala-ala-ala-ala (séq. d'id. n°1), try-gly-gly-phe-met (séq. d'id. n°2), ala-ala-ala-ala-ala (séq. d'id. n°3) et ala-ala-ala-ala-ala-ala (séq. d'id. n°4), mais ne comprenant pas ala-ala, ala-glu et ala-gly ; une activité d'hydrolyse vis-à-vis du lysyl-p-nitroanilide avec un optima de température de 40°C ± 5°C et un optima de pH de 7 ± 0,5 ; une sensibilité aux deux agents chélatants EDTA et 1,10-phénanthroline et aux agents bloquant les groupes sulfhydryle tels que le p-chloromercuribenzoate,le p-chloromercuribenzènesulfonate et le phénoxyacétate d'O-(3-hydroxymercuri-2-méthoxypropyl)carbamoyle, et par la séquence d'acides aminés suivante à l'extrémité N-terminale : ala-val-lys-arg-leu-ile-glu-thr-phe-val-pro-glu-asn-tyr (séq. d'id. n°5).

**2.** Aminopeptidase selon la revendication 1, caractérisée par la séquence d'acides aminés suivante à l'extrémité N-terminale : ala-val-lys-arg-leu-ile-glu-thr-phe-val-pro-glu-asn-tyr-lys-ile-phe-leu-asp-ile-asp-arg-lys (séq. d'id. n°6).

**3.** Aminopeptidase selon la revendication 1, caractérisée par la séquence d'acides aminés suivante à l'extrémité N-terminale : ala-val-lys-arg-leu-ile-glu-thr-phe-val-pro-glu-asn-tyr-lys-ile-phe-leu-asp-ile-asp-arg-lys-Xaa-thr-lys-ile-lys-gly-gln-val-ala-ile (séq. d'id. n°7), dans laquelle Xaa est un acide aminé non déterminé jusqu'à présent.

**4.** Aminopeptidase selon l'une quelconque des revendications 1-3, provenant de lactococcus.

**5.** Aminopeptidase selon la revendication 4, provenant du lactococcus Lactococcus lactis.

**6.** Aminopeptidase selon la revendication 4, provenant du lactococcus Lactococcus lactis. ssp. cremoris.

**7.** Aminopeptidase selon la revendication 4, provenant du lactococcus Lactococcus lactis. ssp. cremoris Wg2.

**8.** Aminopeptidase selon l'une quelconque des revendications 1-7, obtenue par isolement à partir d'une masse cellulaire, d'un milieu de culture ou d'un produit de séparation de bactéries lactiques, dans lesquels l'aminopeptidase est exprimée naturellement, ou à partir d'une masse cellulaire, d'un milieu de culture ou d'un produit de séparation d'un organisme qui, par des techniques de génie génétique, a aquis la capacité d'exprimer l'aminopeptidase.

**9.** Procédé de préparation d'une aminopeptidase selon l'une quelconque des revendications 1-8, caractérisé en ce que l'on soumet un extrait acellulaire brut d'un organisme, en particulier d'un micro-organisme, dans lequel l'aminopeptidase est exprimée, à une ou plusieurs étapes de chromatographie sur colonne à échange d'ions et de filtration sur gel, procédé dans lequel, à partir de chaque éluat recueilli par fractions, on sélectionne une ou plusieurs des fractions ayant une activité hydrolytique vis-à-vis du lysyl-p-nitroanilide.

**10.** Utilisation d'une aminopeptidase selon l'une quelconque des revendications 1-8, pour produire des anticorps monoclonaux ou polyclonaux ayant une affinité et/ou une spécificité pour l'aminopeptidase.

**11.** Procédé pour la préparation de produits alimentaires tels que des fromages et d'autres produits laitiers, des produits à base de viande et des hydrolysats protéiques, en utilisant une aminopeptidase selon l'une quelconque des revendications 1-8.